# EUROPÄISCHE PATENTSCHRIFT

(11) **EP 3 374 778 B1**
(45) Veröffentlichungstag und Bekanntmachung des Hinweises auf die Patenterteilung: **04.09.2019**
(21) Anmeldenummer: 16785451.2
(22) Anmeldetag: 21.10.2016
(51) Int. Cl.: G01R 31/327, G01R 31/36, H01H 71/12, G01R 31/385

(54) **BATTERIEBETRIEBENES RELAISTESTGERÄT**
BATTERY-OPERATED RELAY TEST DEVICE
APPAREIL D'ESSAI À RELAIS ALIMENTÉ PAR ACCUMULATEUR

(30) Priorität: 10.11.2015 AT 509562015
(43) Veröffentlichungstag der Anmeldung: 19.09.2018
(73) Patentinhaber: Omicron electronics GmbH, 6833 Klaus (AT)
(72) Erfinder: KLAPPER, Ulrich, 6830 Rankweil (AT)
(74) Vertreter: Patentanwälte Pinter & Weiss OG
(86) Internationale Anmeldenummer: PCT/EP2016/075347
(87) Internationale Veröffentlichungsnummer: WO 2017/080789

(56) Entgegenhaltungen:
- US-A1- 2003 160 619

## Beschreibung

Die gegenständliche Erfindung betrifft ein Verfahren und eine Testeinrichtung zum Prüfen eines Schutzrelais, wobei in der Testeinrichtung ein Signal erzeugt wird und das Signal an das Schutzrelais angelegt wird. Ebenso wird eine Testanordnung der Testeinrichtungen beschrieben.

Auf dem Gebiet von energietechnischen Anlagen, insbesondere elektrischen Energieübertragungsnetzen, werden Schutzrelais zur Überwachung der Anlage (primäres System) eingesetzt. Um die realen, primären Ströme und Spannungen besser handhaben zu können, werden die Ströme durch Stromwandler und die Spannungen durch Spannungswandler in kleinere, leichter handhabbare Sekundärgrößen transformiert, die im Schutzrelais verarbeitet werden. Trotzdem ist das Schutzrelais zu jedem Zeitpunkt über den Zustand der primären Strom- und Spannungsgrößen im Bilde. Nach verschiedensten Kriterien können Schutzrelais ermitteln, ob im primären System ein Fehler vorliegt und geben dann, je nach Fehler, sofort oder nach einer definierten Verzögerungszeit, ein Ausschaltkommando an einen oder mehrere Leistungsschalter ab, um den Fehlerzustand in der Anlage zu beenden. Mehrere Schutzrelais arbeiten möglichst so zusammen, dass Fehler schnell, sicher aber auch selektiv abgeschaltet werden. Selektiv bedeutet, dass möglichst nur der Teil des Energieübertragungsnetzes, in dem der Fehler aufgetreten ist, abgeschaltet wird, um möglichst viele andere Teile des Energieübertragungsnetzes ungestört weiterbetreiben zu können.

Eine Funktion eines Schutzrelais ist der Überstrom-Zeitschutz. Dabei wird bei Überschreiten des Nennstroms je nach Höhe des Stroms verschieden schnell das Ausschaltkommando abgesetzt. Aus Sicherheitsgründen ist es notwendig oder gefordert, Sicherheitseinrichtungen eines elektrischen Energieübertragungsnetzes, wie beispielsweise das Schutzrelais, in regelmäßigen Abständen auf korrekte Funktion zu prüfen.

Die Prüfung eines Schutzrelais mit Überstrom-Zeitschutz Funktion kann beispielsweise erfolgen indem man in das Schutzrelais einen Prüfstrom, ein- oder dreiphasig, einspeist und die Reaktion des Schutzrelais beobachtet. Testeinrichtungen zur Prüfung von Schutzrelais werden auch "Relaistestgeräte" genannt. Üblicherweise wird das Schutzrelais dazu vom elektrischen Energieübertragungsnetz getrennt und direkt an eine Testeinrichtung angeschlossen und es werden Sekundärgrößen über einen Stromwandler eingespeist. Es sind jedoch auch direkte Prüfungen der Primärgrößen möglich. Es wird überprüft ob das Schutzrelais korrekterweise Strömen unter einer Stromschwelle, beispielsweise bei Nennströmen, nicht auslöst, und wie schnell das Schutzrelais bei verschiedenen Fehlerzuständen auslöst. Beim Überstrom-Zeitschutz gilt üblicherweise, dass mit zunehmender Stromhöhe schneller ausgeschaltet werden soll. Die Testeinrichtung ist mit einem Eingang versehen, der mit dem Leistungsschalterausgang des Schutzrelais verbunden wird und ausgelegt ist, zu registrieren, wann das Schutzrelais schaltet, also den Leistungsschalter schalten würde. Möchte man nun die Signalschwelle, bei der ein Schutzrelais anspricht, ermitteln, so kann ein kleiner Strom so lange kontinuierlich erhöht werden, bis das Schutzrelais reagiert. Solch ein Test kann eine Zeit von mehr als wenigen Sekunden in Anspruch nehmen, ja sogar Minuten dauern.

Da diese Prüfung in der Regel im Feld vor Ort stattfindet und dort nicht immer oder zumindest nicht immer einfach eine Steckdose verfügbar ist, wird die Testeinrichtung mitunter über Stromaggregate versorgt. D.h. es muss für den Test ein Stromaggregat mitgeführt werden, was einerseits den Aufwand erhöht und das auch schwierig in der Handhabung ist (Gewicht, Größe, Treibstoffstand, usw.). Insbesondere an schwer zugänglichen Stellen, die nur zu Fuß erreichbar sind, was bei elektrischen Energieübertragungsnetzen nicht unüblich ist, stellt diese Immobilität einen schwerwiegenden Nachteil dar.

Die US 2003/0160619 A1 behandelt eine Testeinrichtung und einen zu testenden circuit breaker, welcher eine Last mit einer Leitung verbindet. Es wird durch die Testvorrichtung ein Signal, das einen Lichtbogen nachbildet, erzeugt und an die Sicherung angelegt. Damit kann getestet werden, ob der circuit breaker bei Auftreten eines Lichtbogens auch tatsächlich die Last von der Leitung trennt. Weitere Geräte sind aus US3894284, US4351013 und US6618649 bekannt.

Ziel der vorliegenden Erfindung ist es somit, eine Testeinrichtung anzugeben, die effizienter und einfacher zu handhaben ist und die oben beschriebenen Nachteile verringert.

Dieses Ziel wird durch ein Verfahren und eine Vorrichtung erreicht, die dadurch gekennzeichnet sind, dass ein in der Testeinrichtung befindlicher Signalgenerator ein Signal als Pulse mit Pausenzeiten ausgibt, wobei sich die Pulse des Signals und die Pausenzeiten über die Zeit abwechseln, in den Pausenzeiten die Höhe des Signals abgesenkt wird und zumindest ein Puls eine höhere Amplitude als zumindest einer der vorangegangenen Pulse. Die Testeinrichtung ist akkumulatorbetrieben und wird von einem Akkumulator mit einer Versorgungsspannung versorgt, der damit elektrische Energie zur Erzeugung der Pulse zur Verfügung stellt.

Weiter wird das Ziel durch eine Testanordnung erreicht, in der eine Testeinrichtung mit einem Schutzrelais verbunden ist, und einen Signalausgang aufweist, über den ein Signal an einen Signaleingang des Schutzrelais ausgegeben wird, und einen Reaktionseingang aufweist, der mit dem Schaltausgang des Schutzrelais verbunden ist.

Durch die Verwendung eines Akkumulators ist eine größere Mobilität und Flexibilität gewährleistet und es kann auf treibstoffversorgte Stromaggregate oder Notstromaggregate verzichtet werden. Im Betrieb wird der Akkumulator in kurzer Zeit sehr stark belastet, insbesondere dann, wenn Rampen zur Ermittlung von Signalschwellen wie oben beschrieben gefahren werden müssen, und die Prüfung relativ lange dauert. Um eine Belastung des Akkumulators gering zu halten, gibt der Signalgenerator das Signal als Pulse mit Pausenzeiten aus, wobei die Amplituden der Pulse monoton steigend sein können, insgesamt jedenfalls eine steigende Tendenz aufweisen müssen um eine Schaltschwelle zu erreichen. Steigende Tendenz bedeutet, dass zwischen den Pulsen mit steigenden Amplituden auch weitere Pulse, die eine geringere Amplitude als der Tendenz entsprechend aufweisen, auftreten können. Da das Signal in Form einzelner Pulse generiert wird, wird die mittlere benötigte Energie reduziert und der Akkumulator geschont. Das erlaubt trotz der für den Test benötigten, an das elektrischen Energieübertragungsnetz angepassten, Spannungen und (in der Regel sehr hohen) Stromgrößen die Verwendung kleinerer, kompakterer Akkumulatoren, was beispielsweise für ein tragbares Gerät wichtig ist.

Das Signal kann beispielsweise einen Strom oder eine Spannung darstellen, das Verfahren ist auch auf andere Signale anwendbar.

Der Signalgenerator kann eine Spannungsquelle und/oder eine Stromquelle beinhalten.

Weiter kann die Testeinrichtung eine erste Anzahl an Signalausgängen aufweisen, die die erste Anzahl an Signalen erzeugen.

Auch kann die Testeinrichtung eine zweite Anzahl Reaktionseingänge aufweisen.

Vorteilhaft können drei Stromausgänge und drei Spannungsausgänge an der Testeinrichtung vorgesehen sein um die Signale eines dreiphasigen Abzweigs im Energienetz abbilden zu können. Damit kann ein Dreiphasennetz simuliert werden und ein dreiphasiges Schutzrelais überprüft werden. Die Signale der einzelnen Phasen müssen dabei aber nicht unbedingt dieselbe Amplitude aufweisen. Eine Phasenverschiebung von 120° zwischen den Phasen ist üblich, kann im Fehlerfall aber auch gänzlich abweichen. Vorteilhaft können an der Testeinrichtung zudem zwei Reaktionseingänge vorhanden sein, um verschiedene Reaktionen des Schutzrelais erfassen zu können wie beispielsweise ein Auslösen oder eine Anregung. Eine Anregung kann bedeuten, dass eine Signalschwelle kurz überschritten wurde, jedoch nicht lange genug, um ein Auslösen hervorzurufen.

Das Schutzrelais kann, nachdem das Signal eine Signalschwelle erreicht, innerhalb einer Ansprechzeit schalten, wobei von der Testeinrichtung die Höhe des Signals, bei Erreichen der Signalschwelle ermittelt wird.

Dabei ist zu beachten, dass die jeweiligen Pulsdauern die Ansprechzeit des Schutzrelais erreichen, um eine korrekte Funktion des Schutzrelais prüfen zu können. Die zu wählende Dauer der Pausenzeiten ist von der Energie der Pulse abhängig, d.h. von der Amplitude und wiederum der Pulsdauer. Die Ansprechzeit des Schutzrelais ist bei hohen zu schaltenden Signalen tendenziell niedriger vorgesehen, als bei niedrigeren Signalen.

Ganz besonders vorteilhaft ist die zusätzliche Ermittlung der Ansprechzeit t vom Erreichen der Signalschwelle bis zum Schalten des Reaktionsausgangs.

Die Amplituden der Pulse des Signals können über die Zeit um eine, vorzugsweise feste, Signaldifferenz ansteigen. Damit kann das Signal stückchenweise an die Signalschwelle angenähert werden und beispielsweise ein Überstrom-Zeitschutz überprüft werden.

Die Pausenzeiten können variabel sein und von der Amplitude der Pulse des Signals zu einem Zeitpunkt abhängen.

Dies kann beispielsweise durch eine Pulsschwelle erreicht werden, bei der die Pausenzeiten um einen Faktor k erhöht werden. Somit würde sich ab der Pulsschwelle eine andere Steigung der Einhüllenden des Signals ergeben. Es ist auch vorstellbar, dass die Pausenzeiten beispielsweise durch mehrere Pulsschwellen beeinflusst werden, oder auf eine andere Art variabel sind. Mit veränderlichen Pausenzeiten kann erreicht werden, dass dem Akkumulator ab größeren Strömen mehr Zeit für die "Erholung" zur Verfügung steht. Ebenso könnte die Signaldifferenz variabel ausgestaltet sein.

Das Signal kann vorteilhafterweise in den Pausenzeiten auf einen Wert kleiner 1% des vorhergehenden Pulses, vorzugsweise auf Null abgesenkt werden. Dies minimiert die mittlere Leistungsaufnahme aus dem Akkumulator.

Vorteilhafterweise kann der Akkumulator eine Energiedichte von mindestens 500 J/g aufweisen. Vorteilhafterweise kann der Akkumulator oder ein Teil davon auf Lithium-Ionen- oder Lithium-Polymer-Basis aufgebaut sein.

Ebenso kann die Testeinrichtung portabel ausgestaltet sein, wobei das geringe Gewicht durch Nutzung eines Akkumulators für die Verwendung im Feld besonders vorteilhaft ist.

Es kann eine Anpassungseinrichtung mit der Versorgungsspannung versorgt werden und weiter den Signalgenerator mit der Zwischenspannung versorgen.

Damit ist es beispielsweise möglich eine hohe Versorgungsspannung eines Akkumulators in eine niedrigere Zwischenspannung zu transformieren, wogegen der vom Akkumulator gelieferte Strom in einen höheren Strom transformiert werden kann um somit den Signalgenerator zu versorgen. Dies ist vorteilhaft, da der Signalgenerator in der Regel hohe Ströme benötigt, selbstverständlich kann jedoch auch eine niedrige Versorgungsspannung des Akkumulators in eine hohe Zwischenspannung transformiert werden und ein vom Akkumulator gelieferter hoher Strom in einen niedrigen Strom transformiert werden.

Die Anpassungseinrichtung muss möglicherweise mit hohen Taktfrequenzen arbeiten, daher sind zusätzliche Tiefpassfilter zum Unterdrücken der entstehenden Störungen vorteilhaft.

Diese Anpassungseinrichtung kann einen Step-Up-Konverter und/oder einen Step-Down-Konverter beinhalten.

Vorteilhafterweise kann zumindest ein Teil der Anpassungseinrichtung und/oder zumindest ein Teil des Signalgenerators mittels einer Notausschaltung bedarfsweise deaktiviert werden Da die von der Anpassungseinrichtung erzeugten Ströme sehr hoch sein können, wäre es schwierig diese sicher zu trennen. Daher wird zumindest ein Teil der Anpassungseinrichtung, vorteilhafterweise vorhandene Leistungselektronik, gezielt deaktiviert, wobei eine Redundanz der deaktivierten Teile die nötige Sicherheit gewährleistet. Diese Redundanz kann beispielsweise dadurch erzielt werden, indem Anpassungseinrichtung und Signalgenerator deaktiviert werden.

Die Form des Signals kann von einer Steuereinheit bestimmt werden, wobei das Ergebnis der Steuereinheit von einem Digital/Analog-Wandler zur Realisierung des Signals verarbeitet wird und der Digital/Analog-Wandler den Signalgenerator ansteuert.

Die gegenständliche Erfindung wird nachfolgend unter Bezugnahme auf die Figuren 1 bis 5 näher erläutert, die beispielhaft, schematisch und nicht einschränkend vorteilhafte Ausgestaltungen der Erfindung zeigen. Dabei zeigt
Fig.1 ein Schutzrelais 2 in einem Versorgungsnetz 6
Fig.2 ein Schutzrelais 2, welches mit einer Testeinrichtung 4 verbunden ist
Fig.3 möglicher Aufbau einer Testeinrichtung 4
Fig.4 den Verlauf eines Signals S mit fixen Pausenzeiten τ₁= τ₂= τ₃= τ₄= τ₅
Fig.5 den Verlauf eines Signals S mit einer Pulsschwelle S₁
Fig.6 den Verlauf eines Signals S mit streng monoton steigenden Pausenzeiten τ₁ < τ₂< τ₃< τ₄< τ₅

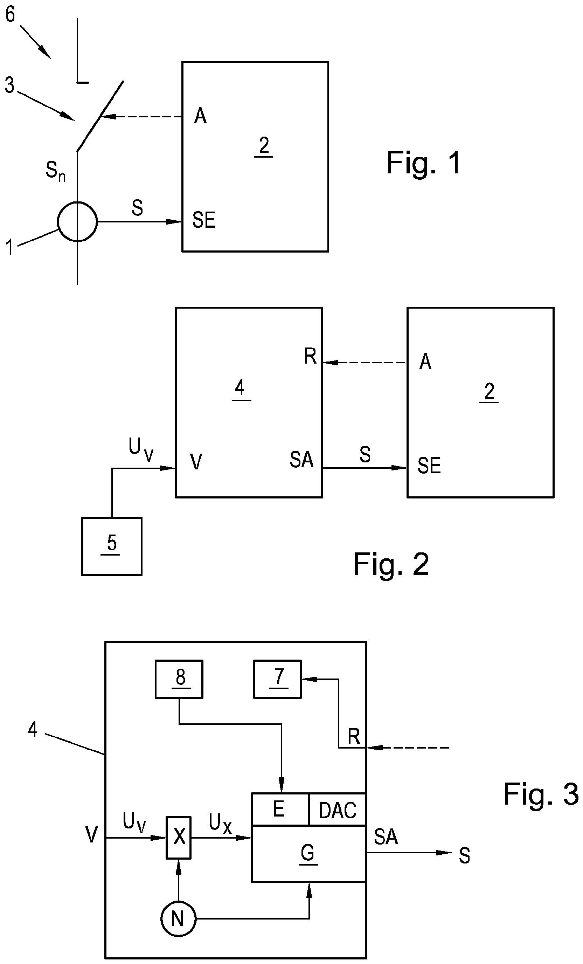

In Fig. 1 ist ein Schutzrelais 2 über den Signaleingang SE und den Schaltausgang A mit dem elektrischen Energieversorgungsnetz 6 verbunden. Das elektrische Energieversorgungsnetz 6 kann auch ein Leitungsabschnitt oder ein Leitungsabzweig eines größeren Versorgungsnetzes sein. Ein optional vorhandener Signalwandler 1 misst ein Vorsignal Sₙ (Primärgröße) -wenn das Signal durch einen Strom repräsentiert wird, ist der Signalwandler 1 in der Regel als Stromwandler oder Stromsensor ausgestaltet- des Energieversorgungsnetzes 6 und transformiert dieses in ein Signal S (Sekundärgröße), welches dem Schutzrelais 2 über den Signaleingang SE zugeführt wird. Beispielsweise in Niederspannungsnetzen ist es auch möglich das Vorsignal Sₙ direkt dem Schutzrelais zuzuführen. Beispielsweise bei einer Funktion als Überstrom-Zeitschutz ist das Schutzrelais 2 so ausgelegt, dass es den Schaltausgang A schaltet, und somit den damit verbundenen Leistungsschalter 3 des elektrischen Energieversorgungsnetzes 6 öffnet, sobald eine bestimmte, voreingestellte Signalschwelle S_{S} für eine festgelegte Zeitdauer überschritten wird. Somit wird der elektrische Kreis des Energieversorgungsnetzes 6 (oder des jeweiligen Netzsegments) unterbrochen, womit beispielsweise im elektrischen Energieversorgungsnetz 6 ein Schutz vor Überströmen gewährleistet wird.

Um die Signalschwelle S_{S}, bei der das Schutzrelais 2 tatsächlich schaltet, zu bestimmen, wird das Schutzrelais 2 für eine Funktionsprüfung aus dem Energieversorgungsnetz 6 abgeklemmt und mit einer Testeinrichtung 4 verbunden, wie in Fig.2 dargestellt. Die Testeinrichtung 4 weist einen Signalausgang SA und einen Reaktionseingang R auf. Für die Funktionsprüfung wird die Verbindung vom Schutzrelais 2 zum Signalwandler 1 (bzw. falls kein Stromwandler vorhanden ist die Verbindung zum Energieversorgungsnetz 6) und zum Leistungsschalter 3 unterbrochen und der Signalausgang SA der Testeinrichtung 4 mit dem Signaleingang SE des Schutzrelais 2, sowie der Schaltausgang A des Schutzrelais 2 mit dem Reaktionseingang R der Testeinrichtung 4verbunden. Die Testeinrichtung 4 wiederum wird von einem Akkumulator 5, der vorzugsweise in der Testeinrichtung 4 integriert ist, über einen Versorgungseingang V mit einer Versorgungsspannung U_{V} versorgt. Es wird zum Prüfen des Schutzrelais 2, ein Signal S von der Testeinrichtung 4 an das Schutzrelais 2 angelegt.

Wenn der Schutz beispielsweise einen Überstrom-Zeitschutz umfasst, schaltet das Schutzrelais 2 innerhalb einer Ansprechzeit t_{A}, nachdem das Signal S die zu eruierende Signalschwelle Sₛ erreicht hat. Von der Testeinrichtung 4 wird die Höhe, also die Amplitude des Signals S ermittelt, bei deren Erreichen das Schutzrelais 2 anspricht.

Dazu ist in der Testeinrichtung 4 eine Auswerteeinheit 7 vorgesehen, die mit dem Reaktionseingang R verbunden ist und einen Schaltimpuls des Schutzrelais 2, das am Schaltausgang A ausgegeben wird, erfasst.

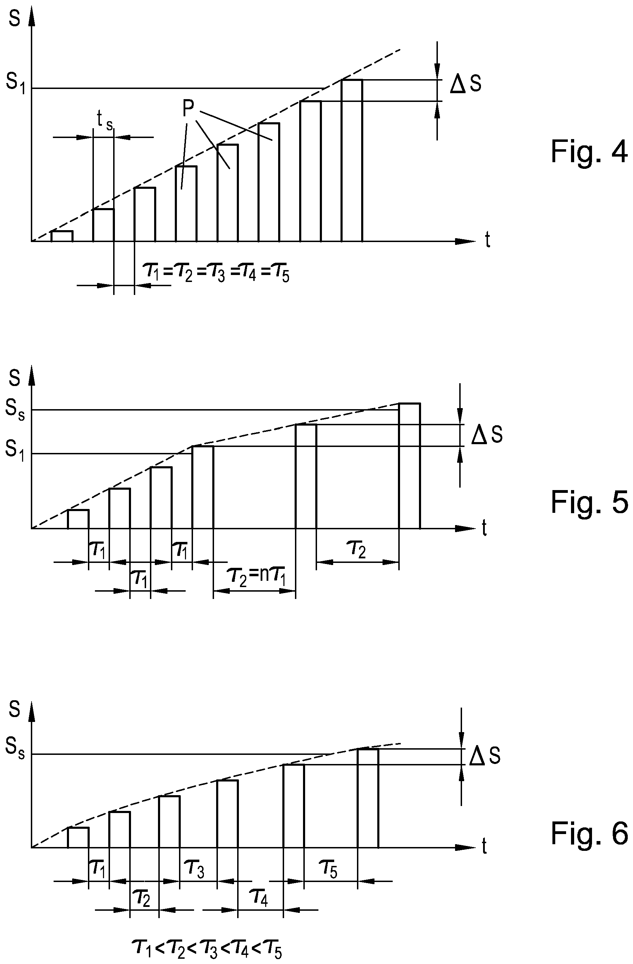

Ein Signalgenerator G gibt das Signal S als Pulse P mit Pausenzeiten τ₁, τ₂, τ₃, τ₄, τ₅ am Signalausgang SA aus, wobei sich die Pulse P des Signals S und Pausenzeiten τ₁, τ₂, τ₃, τ₄, τ₅ über die Zeit t abwechseln (Fig.3). In den Pausenzeiten τ₁, τ₂, τ₃, τ₄, τ₅ wird die Amplitude des Signals S auf einen niedrigen Wert, beispielsweise 1% der vorigen Amplitude oder auch Null, abgesenkt. Zumindest ein Puls P weist eine höhere Amplitude auf als zumindest einer der vorangegangenen Pulse P, um einen ansteigendes Signal S abzubilden, wie in Fig.4 exemplarisch dargestellt. Durch die Realisierung der Pausenzeiten τ₁, τ₂, τ₃, τ₄, τ₅ wird der Akkumulator 5 geschont.

Ganz besonders vorteilhaft ist eine Ausgestaltung, bei der auch die Ansprechzeit t_{A} des Schutzrelais 2 von der Testeinrichtung 4, vorzugsweise in der Auswerteeinheit 7, ermittelt wird. Die Ansprechzeit t_{A} des Schutzrelais 2 beschreibt also die Zeit vom Erreichen der Signalschwelle S_{S} durch das Signal S bis zum Schalten des Reaktionsausgangs R.

Eine in der Testeinrichtung 4 befindliche Anpassungseinrichtung X kann die Versorgungsspannung Uᵥ des Akkumulators 5 in eine Zwischenspannung U_{X} wandeln, welche wiederum den Signalgenerator G versorgt, wie auch in Fig.3 dargestellt.

Die Anpassungseinrichtung X kann dazu dienen, hohe Spannungen in niedrige Spannungen und niedrige Ströme in hohe Ströme zu wandeln, oder auch umgekehrt.

Diese Anpassungseinrichtung X kann einen Step-Up-Konverter und/oder einen Step-Down-Konverter beinhalten.

Weiters kann zumindest ein Teil der Anpassungseinrichtung X und/oder des Signalgenerators G mittels einer Notausschaltung N bedarfsweise deaktiviert werden.

Dieser Teil der Anpassungseinrichtung X kann beispielsweise Leistungselektronik umfassen, die Teil einer Konverterschaltung ist. Da hohe Ströme schwer sauber zu trennen sind, ist eine Möglichkeit eine Notausschaltung N zu realisieren, das gezieltes Deaktivieren von (redundanten) Schaltungsteilen, wie z.B. der Leistungselektronik.

Die Testeinrichtung 4, bzw. der Signalgenerator G, kann eine Spannungsquelle und/oder eine Stromquelle beinhalten und ein Signal S als Spannung oder Strom erzeugen.

Zudem kann die Form des Signals S von einer Steuereinheit E errechnet werden, wobei das Ergebnis der Steuereinheit E von einem Digital/Analog-Wandler DAC zur Realisierung des Signals S verarbeitet wird und der Digital/Analog-Wandler DAC den Signalgenerator G ansteuert.

Dazu kann in der Testeinrichtung 4 eine mit der Steuereinheit E verbundene Eingabeeinheit 8 vorgesehen sein, über die z.B. ein bestimmter durchzuführender Test eingestellt werden kann. Die Steuereinheit E und der Digital/Analog-Wandler DAC können sich dabei im Signalgenerator G befinden.

Weiters kann der Signalgenerator G n>1 Signalausgänge aufweisen, die n Signale Sₙ erzeugen um ein Schutzrelais 2 eines Mehrphasennetzes für alle n Phasen gleichzeitig testen zu können.

Vorteilhafterweise ist n= 3, womit ein Dreiphasennetz simuliert werden kann. Damit kann ein dreiphasiges Schutzrelais 2 überprüft werden. Die n Signale Sₙ müssen dabei aber nicht unbedingt gleich sein.

Weiters kann die Testeinrichtung (4) eine zweite Anzahl an Reaktionseingängen R aufweisen um verschiedene Reaktionen des Schutzrelais (2) zu erfassen, wie beispielsweise ein Auslösen oder eine Anregung.

Ein Signal S wird in einer bestimmten Höhe (Amplitude) über eine Pulsdauer t_{S} erzeugt und nach Ablauf der Pulsdauer t_{S} für eine Pausenzeit τ₁, τ₂, τ₃, τ₄, τ₅ abgesenkt. Pausenzeiten τ₁, τ₂, τ₃, τ₄, τ₅ in der Größenordnung von 500ms bis 1s sind dabei die Regel. Die Länge der Pulsdauer t_{S} muss dabei zumindest so groß wie die Ansprechzeit t_{A} des Schutzrelais 2 sein, da ansonsten die korrekte Funktion des Schutzrelais 2 nicht getestet werden kann. Dabei ist in den meisten Fällen zumindest eine Pulsdauer t_{S} von 10ms erforderlich, übliche Pulsdauern t_{S} betragen in etwa 30ms, es sind jedoch auch Pulsdauern im Sekundenbereich möglich. Ausschlaggebender Faktor ist hierbei die Ansprechzeit t_{A} des Schutzrelais 2, welche wiederum von der Höhe des zu schaltenden Signals abhängt. Ein höherer Strom muss in der Regel schneller, also mit einer kürzeren Ansprechzeit t_{A} geschaltet werden, als ein niedrigerer Strom.

Die Pulsdauer t_{S} ist in den Figuren 3 bis 5 als Konstante dargestellt, kann jedoch auch, beispielsweise abhängig von der Höhe des Signals S, variieren. Dies kann beispielsweise genutzt werden, um die Energie eines Pulses P niedrig zu halten, indem mit steigender Amplitude die Pulsdauern t_{S} verringert werden. Nach Ablauf der Pausenzeit τ₁, τ₂, τ₃, τ₄, τ₅ wird das Signal um die Signaldifferenz ΔS erhöht für eine weitere Pulsdauer t_{S} ausgegeben, woraufhin abermals eine Pausenzeit τ₁, τ₂, τ₃, τ₄, τ₅ folgt. Dies erfolgt vorteilhaft so lange, bis das Schutzrelais 2 anspricht oder auslöst. Vorteilhafterweise ist die Signaldifferenz ΔS immer konstant und positiv. Es ist jedoch auch vorstellbar, dass die Signaldifferenz ΔS variabel, bzw. abschnittsweise negativ oder Null ist, was beispielsweise von der aktuellen Höhe des Signals S abhängen kann. Um die Signalschwelle S_{S} zu erreichen, muss jedoch zumindest ein Puls P eine höhere Amplitude als zumindest einer der vorangehenden Pulse P aufweisen, es sei denn die Amplitude des ersten Pulses P des Signals S erreicht die Signalschwelle S_{S}. In diesem Fall schaltet das Schutzrelais 2 sofort.

Die zwischen den einzelnen Pulsen P des Signals S andauernden Pausenzeiten τ₁, τ₂, τ₃, τ₄, τ₅ des Signals S können immer die gleiche Länge aufweisen, jedoch auch von der aktuellen Amplitude des Signals S oder einem anderen Faktor abhängen.

Da die Wahl der Pausenzeiten τ₁, τ₂, τ₃, τ₄, τ₅ vorzugsweise von der gewählten Pulsdauer t_{S} abhängt, kann also sowohl auf variable Pulsdauern t_{S} reagiert werden, andererseits auch die mittlere Energie der Pulse P z.B. abschnittweise gesenkt werden. Eine geringere Energieaufnahme der Testeinrichtung 4 und damit eine geringere Energieentnahme aus dem Akkumulator 5 bewirkt eine Schonung des Akkumulators 5.

In Fig.4 ist ein beispielhafter Verlauf eines Signals S über die Zeit t dargestellt. Die strichliert dargestellte Einhüllende der von Pausenzeiten τ₁, τ₂, τ₃, τ₄, τ₅ unterbrochenen Pulse des Signals S deutet das ansteigende Signal S an, wobei in diesem Beispiel die Pausenzeiten τ₁, τ₂, τ₃, τ₄, τ₅ konstant sind und die Höhe der aufeinander folgenden Pulse P des Signals S bei konstanter Signaldifferenz ΔS linear steigt.

Es ist auch ein Verlauf nach Fig. 5 möglich, bei dem die Pausenzeiten τ, τ₁ sobald die Amplitude des aktuellen Pulses P des Signals S eine Pulsschwelle S₁ erreicht, erhöht werden. Damit ergibt sich bei konstanter Signaldifferenz ΔS die als strichliert dargestellte Einhüllende in Form eines ansteigenden Signals S, wobei nach Erreichen einer Pulsschwelle S₁ die Steigung des Signals S reduziert wird. Der Vorteil einer Erhöhung der Pausenzeiten bei zunehmender Amplitude liegt darin, dass die mittlere Akkubelastung mit zunehmender Amplitude nicht zunehmen muss, da die längeren Pausen den zunehmenden Leistungsbedarf für die Pulse kompensieren können.

In den Pausenzeiten τ₁, τ₂, τ₃, τ₄, τ₅ wird die Höhe des Signals S wie erwähnt reduziert. Vorteilhafterweise kann das Signal S in den Pausenzeiten τ₁, τ₂, τ₃, τ₄, τ₅ auf einen Wert kleiner als 1% des vorhergehenden Pulses P, oder auch auf Null gesetzt werden, wie in den Fig.3-5 dargestellt, was die Laufzeit des Akkumulators 5 verlängern kann.

Vorteilhafterweise kann der Akkumulator 5 eine Energiedichte von mindestens 500 J/g aufweisen.

Vorteilhafterweise steigen die Pausenzeiten τ₁, τ₂, τ₃, τ₄, τ₅ mit steigender Höhe des Signals S kontinuierlich an. Die Pausenzeiten τ₁, τ₂, τ₃, τ₄, τ₅ können somit von Puls P zu Puls P streng monoton steigend sein, wodurch sich für das Signal S eine strichliert dargestellte Einhüllende mit über die Zeit t reduzierter Steigung ergibt. Diese Ausgestaltung ist in Fig. 5 ebenfalls mit konstanter Signaldifferenz ΔS dargestellt.

Es ist natürlich auch vorstellbar, dass die Pausenzeiten τ₁, τ₂, τ₃, τ₄, τ₅, (z.B. abschnittweise) reduziert werden, oder abschnittsweise gleich bleiben.

Selbstverständlich sind auch Mischvarianten der soeben genannten Verläufe, sowie weitere Variationen der Pausenzeiten τ₁, τ₂, τ₃, τ₄, τ₅, sowie der Signaldifferenz ΔS abhängig von der aktuellen Amplitude des Pulses P möglich. So können beispielsweise mehrere Pulsschwellen S₁ vorhanden sein und die Signaldifferenz ΔS und/oder die Pausenzeiten τ₁, τ₂, τ₃, τ₄, τ₅ mehrmals geändert werden.

Die Testeinrichtung 4 kann durch das geringe Gewicht durch Nutzung eines Akkumulators 5 portabel ausgestaltet sein, was für die Verwendung im Feld besonders vorteilhaft ist.

## Patentansprüche

1. Verfahren zum Prüfen eines Schutzrelais (2), wobei in einer Testeinrichtung (4) ein Signal (S), vorzugsweise eine Spannung oder ein Strom, erzeugt wird und das Signal (S) an das Schutzrelais (2) angelegt wird, **dadurch gekennzeichnet, dass** ein in der Testeinrichtung (4) befindlicher Signalgenerator (G) das Signal (S) als Pulse (P) mit Pausenzeiten (τ₁, τ₂, τ₃, τ₄, τ₅) ausgibt, wobei sich die Pulse (P) des Signals (S) und die Pausenzeiten (τ₁, τ₂, τ₃, τ₄, τ₅) über die Zeit (t) abwechseln, in den Pausenzeiten (τ₁, τ₂, τ₃, τ₄, τ₅) die Höhe des Signals (S) abgesenkt wird und zumindest ein Puls (P) eine höhere Amplitude als zumindest einer der vorangegangenen Pulse (P) aufweist, **und dass** die Testeinrichtung (4) akkumulatorbetrieben ist und von einem Akkumulator (5) mit einer Versorgungsspannung (Uᵥ) versorgt wird, der damit elektrische Energie zur Erzeugung der Pulse (P) zur Verfügung stellt.

2. Verfahren nach Anspruch 1, **dadurch gekennzeichnet, dass** das Schutzrelais (2) nachdem das Signal (S) eine Signalschwelle (Sₛ) erreicht innerhalb einer Ansprechzeit (t_{A}) schaltet, und von der Testeinrichtung (4) die Höhe des Signals (S), bei Erreichen der Signalschwelle (Sₛ) ermittelt wird, und vorzugsweise die Ansprechzeit (t_{A}) ermittelt wird

3. Verfahren nach Anspruch 1 oder 2, **dadurch gekennzeichnet, dass** das die Amplituden der Pulse (P) des Signals (S) über die Zeit t um eine, vorzugsweise feste, Signaldifferenz ΔS ansteigen.

4. Verfahren nach einem der Ansprüche 1 bis 3, **dadurch gekennzeichnet, dass** die Pausenzeiten (τ₁, τ₂, τ₃, τ₄, τ₅) von der Amplitude der Pulse (P) des Signals (S) zum Zeitpunkt t abhängen.

5. Verfahren nach einem der Ansprüche 1 bis 4, **dadurch gekennzeichnet, dass** das Signal (S) in den Pausenzeiten kleiner 1% des vorhergehenden Pulses (P), vorzugsweise Null, wird.

6. Verfahren nach einem der Ansprüche 1 bis 5, **dadurch gekennzeichnet, dass** zumindest ein Teil des Signalgenerators (G) mittels einer Notausschaltung (N) bedarfsweise deaktiviert wird.

7. Verfahren nach einem der Ansprüche 1 bis 6, **dadurch gekennzeichnet, dass** die Form des Signals (S) von einer Steuereinheit (E) bestimmt wird und das Ergebnis der Steuereinheit (E) von einem Digital/Analog-Wandler (DAC) zur Realisierung des Signals (S) verarbeitet wird und der Digital/Analog-Wandler (DAC) den Signalgenerator (G) ansteuert.

8. Testeinrichtung zum Prüfen eines Schutzrelais (2), welche einen Signalausgang (SA) aufweist, über den ein Signal (S) ausgegeben wird, **dadurch gekennzeichnet, dass** die Testeinrichtung (4) einen Signalgenerator (G), der vorzugsweise eine Spannungsquelle und/oder eine Stromquelle beinhaltet, beinhaltet, der ausgestaltet ist, das Signal (S) als Pulse (P) mit Pausenzeiten (τ₁, τ₂, τ₃, τ₄, τ₅) auszugeben, wobei sich die Pulse (P) des Signals (S) und Pausenzeiten (τ₁, τ₂, τ₃, τ₄, τ₅) über die Zeit (t) abwechseln, in den Pausenzeiten (τ₁, τ₂, τ₃, τ₄, τ₅) die Höhe des Signals (S) abgesenkt wird und zumindest ein Puls (P) eine höhere Amplitude als zumindest einer der vorangegangenen Pulse (P) aufweist, **und dass** die Testeinrichtung akkumulatorbetrieben ist und dass ein Akkumulator (5), der vorzugsweise eine Energiedichte von mindestens 500 J/g aufweist, vorgesehen ist, der eine Versorgungsspannung (Uᵥ) für die Testeinrichtung (4) zur Verfügung stellt.

9. Testeinrichtung nach Anspruch 8, **dadurch gekennzeichnet, dass** die Testeinrichtung (4) eine Anpassungseinrichtung (X), die vorzugsweise einen Step-Up-Konverter und/oder einen Step-Down-Konverter beinhaltet, beinhaltet, die die Versorgungsspannung (Uᵥ) in eine Zwischenspannung (Ux) wandelt, welche den Signalgenerator (G) versorgt.

10. Testeinrichtung nach Ansprch 8 oder 9, **dadurch gekennzeichnet, dass** die Testeinrichtung (4) n>1 Signalausgänge (SA) aufweist, die n Signale (S) erzeugen.

11. Testeinrichtung nach einem der Ansprüche 8 bis 10, **dadurch gekennzeichnet, dass** eine Notausschaltung (N) vorhanden ist, die zumindest einen Teil des Signalgenerators (G) bedarfsweise deaktiviert.

12. Testeinrichtung nach einem der Ansprüche 9 oder10, **dadurch gekennzeichnet, dass** eine Notausschaltung (N) vorhanden ist, die zumindest einen Teil der Anpassungseinrichtung (X) bedarfsweise deaktiviert.

13. Testeinrichtung nach einem der Ansprüche 8 bis 11, **dadurch gekennzeichnet, dass** die Testeinrichtung (4) portabel ausgestaltet ist.

14. Testeinrichtung nach einem der Ansprüche 8 bis 13, **dadurch gekennzeichnet, dass** eine Steuereinheit (E) vorgesehen ist, die die Form des Signals (S) bestimmt **und dass** ein Digital/Analog-Wandler (DAC) vorhanden ist, der das Ergebnis der Steuereinheit (E) zur Realisierung des Signals (S) verarbeitet.

15. Testanordnung mit einer Testeinrichtung nach einem der Ansprüche 8 bis 14, wobei die Testeinrichtung (4) mit einem Schutzrelais (2) verbunden ist, und einen Signalausgang (SA) aufweist, über den ein Signal (S) an einen Signaleingang (SE) des Schutzrelais (2) ausgegeben wird, und einen Reaktionseingang (R) aufweist, der mit dem Schaltausgang (A) des Schutzrelais (2) verbunden ist.

## Claims

1. A method for testing a protection relay (2), wherein a signal (S), preferably a voltage or a current, is generated in a test device (4), and the signal (S) is applied to the protection relay (2), **characterized in that** a signal generator (G) provided in the test device (4) outputs the signal (S) as pulses (P) having pause times (τ₁, τ₂, τ₃, τ₄, τ₅), wherein the pulses (P) of the signal (S) and the pause times (τ₁, τ₂, τ₃, τ₄, τ₅) alternate over time (t), the level of the signal (S) is reduced during the pause times (τ₁, τ₂, τ₃, τ₄, τ₅) and at least one pulse (P) has an amplitude which is higher than at least one of the preceding pulses (P), **and that** the test device (4) is accumulator driven and is supplied with a supply voltage (U_{V}) by an accumulator (5), which thus provides electric energy for generating the pulses (P).

2. The method of claim 1, **characterized in that** the protection relay (2) switches within a reaction time (t_{A}) after the signal (S) has reached a signal threshold (Ss), and that the test device (4) determines the level of the signal (S) upon reaching the signal threshold (S_{S}) and preferably the reaction time (t_{A}) is determined.

3. The method of claim 1 or 2, **characterized in that** the amplitudes of pulses (P) of signal (S) increase over time (t), by a preferably fixed signal difference (ΔS).

4. The method of any of claims 1 to 3, **characterized in that** the pause times τ₁, τ₂, τ₃, τ₄, τ₅ depend on the amplitude of pulses (P) of signal (S) at time (t).

5. The method of any of claims 1 to 4, **characterized in that** the signal (S) during the pause times is smaller than 1% of the preceding pulse (P), preferably zero.

6. The method of any of claims 1 to 5, **characterized in that** at least a part of the adaptation device (X) and/or at least a part of the signal generator (G) are deactivated by an emergency-off circuit (N), if necessary.

7. The method of any of claims 1 to 6, **characterized in that** the form of the signal (S) is determined by a control unit (E) and the result of the control unit (E) is processed by a digital/analog converter (DAC) in order to generate signal (S) and the digital/analog converter (DAC) drives the signal generator (G).

8. A test device for testing a protection relay (2), which has a signal output (SA), through which a signal (S) is output, **characterized in that** the test device (4) comprises a signal generator (G), which preferably comprises a voltage source and/or a current source, which is configured for outputting the signal (S) in the form of pulses (P) having pause times (τ₁, τ₂, τ₃, τ₄, τ₅), wherein the pulses (P) of signal (S) and the pause times (τ₁, τ₂, τ₃, τ₄, τ₅) alternate over time (t), the level of signal (S) is reduced during the pause times (τ₁, τ₂, τ₃, τ₄, τ₅) and at least one pulse (P) has an amplitude which is at least higher than one of the preceding pulses (P) **and that** the test device is accumulator driven and an accumulator (5) which preferably has an energy density of at least 500 J/g, is provided, which provides a power voltage (U_{V}) for the test device (4).

9. The test device of claim 8, **characterized in that** the test device (4) comprises an adaptation device (X), which preferably comprises a step-up converter and/or a step-down converter, which converts the power voltage (U_{V}) into an intermediate voltage (Ux), which is supplied to the signal generator (G).

10. The test device claim 8 or 9, **characterized in that** the test device (4) has n > 1 signal outputs (SA), which generate n signals (S).

11. The test device of any of claims 8 to 10, **characterized in that** an emergency-off circuit (N) is provided, which deactivates, if necessary, at least a part of the adaptation device (X) and/or at least a part of the signal generator (G).

12. The test device of claims 9 or 10, **characterized in that** an emergency-off circuit (N) is provided, which deactivates, if necessary, at least a part of the adaptation device (X).

13. The test device of any of claims 8 to 11, **characterized in that** the test device (4) is configured portable.

14. The test device of any of claims 8 to 13, **characterized in that** a control unit (E) is provided, which determines the form of the signal (S) and that a digital/analog converter (DAC) is provided, which processes the result of the control unit (E) in order to generate the signal (S).

15. A test arrangement having a test device of any of claims 9 to 14, wherein the test device (4) is connected to a protection relay (2), and has a signal output (SA), through which a signal (S) is supplied to a signal input (SE) of the protection relay (2), and has a reaction input (R), which is connected to the switching output (A) of the protection relay (2).

## Revendications

1. Procédé pour tester un relais de protection (2), un signal (S), de préférence une tension ou un courant, étant généré dans un dispositif de test (4), et le signal (S) étant appliqué au relais de protection (2), **caractérisé en ce qu'**un générateur de signal (G) situé dans le dispositif de test (4) délivre le signal (S) sous forme d'impulsions (P) avec des temps de pause (T₁, T₂, T₃, T₄, T₅), les impulsions (P) du signal (S) et les temps de pause (T₁, T₂, T₃, T₄, T5) alternant pendant le temps (t), le niveau du signal (S) étant abaissé dans les temps de pause (T₁, T₂, T₃, T₄, T₅), et au moins une impulsion (P) présentant une amplitude supérieure à au moins une des impulsions (P) précédentes, et **en ce que** le dispositif de test (4) est alimenté par un accumulateur (5) avec une tension d'alimentation (Uᵥ) qui fournit ainsi de l'énergie électrique pour générer les impulsions (P).

2. Procédé selon la revendication 1, **caractérisé en ce que** le relais de protection (2) commute après que le signal (S) a atteint un seuil de signal (Sₛ) dans un temps de réponse (tA), et le niveau du signal (S) est déterminé par le dispositif de test (4) lorsque le seuil de signal (Sₛ) est atteint, et de préférence le temps de réponse (t_{A}) est déterminé

3. Procédé selon la revendication 1 ou 2, **caractérisé en ce que** les amplitudes des impulsions (P) du signal (S) augmentent avec le temps t d'une différence de signal ΔS, de préférence fixe.

4. Procédé selon l'une des revendications 1 à 3, **caractérisé en ce que** les temps de pause (T₁, T₂, T₃, T₄, T₅) dépendent de l'amplitude des impulsions (P) du signal (S) à l'instant t.

5. Procédé selon l'une des revendications 1 à 4, **caractérisé en ce que** le signal (S) dans les temps de pause est inférieur à 1 % de l'impulsion (P) précédente , de préférence zéro.

6. Procédé selon l'une des revendications 1 à 5, **caractérisé en ce que** au moins une partie du générateur de signal (G) est désactivée au moyen d'un circuit d'arrêt d'urgence (N) si nécessaire.

7. Procédé selon l'une des revendications 1 à 6, **caractérisé en ce que** la forme du signal (S) est déterminée par une unité de commande (E) et le résultat de l'unité de commande (E) est traité par un convertisseur numérique-analogique (DAC) pour produire le signal (S) et le convertisseur numérique-analogique (DAC) actionne le générateur de signal (G).

8. Dispositif de test pour tester un relais de protection (2), qui présente une sortie de signal (SA) par l'intermédiaire de laquelle un signal (S) est délivré, **caractérisé en ce que** le dispositif de test (4) contient un générateur de signal (G) qui contient de préférence une source de tension et/ou une source de courant destinée à délivrer le signal (S) en impulsions (P) avec des temps de pause (T₁, T₂, T₃, T₄, T₅), les impulsions (P) du signal (S) et les temps de pause (T₁, T₂, T₃, T₄, T₅) alternant dans le temps (t), le niveau du signal (S) étant abaissé dans les temps de pause (T₁, T₂, T₃, T₄, T₅) et au moins une impulsion (P) ayant une amplitude supérieure à au moins une des impulsions (P) précédentes ; **en ce que** le dispositif de test est alimenté par un accumulateur, et **en ce que**, de préférence avec une densité énergétique de 500 J/g au moins, un accumulateur (5) qui fournit au dispositif de test (4) une tension d'alimentation (Uᵥ) est prévu, lequel fournit une tension d'alimentation.

9. Dispositif de test selon la revendication 8, **caractérisé en ce que** le dispositif de test (4) comprend un dispositif d'adaptation (X), comprenant de préférence un convertisseur élévateur et/ou un convertisseur abaisseur, qui convertit la tension d'alimentation (Uᵥ) en une tension intermédiaire (Uₓ) qui alimente le générateur de signal (G).

10. Dispositif de test selon la revendication 8 ou 9, **caractérisé en ce que** le dispositif de test (4) présente n>1 sorties de signal (SA) qui génèrent n signaux (S).

11. Dispositif de test selon l'une des revendications 8 à 10, **caractérisé en ce qu'**il **e**xiste un circuit d'arrêt d'urgence (N) qui désactive au moins une partie du générateur de signal (G) si nécessaire.

12. Dispositif de test selon l'une des revendications 9 ou 10, **caractérisé en ce qu'**il existe un circuit d'arrêt d'urgence (N) qui désactive au moins une partie du dispositif d'adaptation (X) si nécessaire.

13. Dispositif de test selon l'une des revendications 8 à 11, **caractérisé en ce que** le dispositif de test (4) est conçu pour être portable.

14. Dispositif de test selon l'une des revendications 8 à 13, **caractérisé en ce qu'**il est prévu une unité de commande (E) qui détermine la forme du signal (S) et **en ce qu'**il est équipé d'un convertisseur numérique-analogique (DAC) qui traite le résultat de l'unité de commande (E) pour produire le signal (S).

15. Ensemble de test comprenant un dispositif de test selon l'une des revendications 8 à 14, dans lequel le dispositif de test (4) est relié à un relais de protection (2) et présente une sortie de signal (SA) via laquelle un signal (S) est transmis à une entrée de signal (SE) du relais de protection (2), et présente une entrée de réaction (R) qui est reliée à la sortie de commutation (A) du relais de protection (2).
